# EUROPEAN PATENT APPLICATION

(11) **EP 3 875 619 A1**
(43) Date of publication of application: **08.09.2021**
(21) Application number: 19879402.6
(22) Date of filing: 29.10.2019
(51) Int. Cl.: C22C 1/00, G06F 30/27, G06F 30/10

(54) **MATERIAL EXPLORATION APPARATUS, METHOD, AND PROGRAM**

(30) Priority: 31.10.2018 JP 2018206018
(71) Applicant: Showa Denko K.K., Tokyo 105-8518 (JP)
(72) Inventor: OKUNO, Katsuki, Tokyo 105-8518 (JP); OKUNO, Yoshishige, Tokyo 105-8518 (JP)
(74) Representative: Mewburn Ellis LLP
(86) International application number: PCT/JP2019/042331
(87) International publication number: WO 2020/090805

(57) **Abstract**

Efficiency of a material search is improved. An apparatus according to one embodiment of the present invention includes a composition obtaining unit configured to obtain a candidate composition, a thermodynamic calculation unit configured to perform thermodynamic calculation on the candidate composition, a manufacturing determining unit configured to determine whether the candidate composition is a manufacturable material based on a correspondence relation between a result of the thermodynamic calculation performed on a predetermined composition and manufacturability indicating whether a material having the predetermined composition is manufacturable, and an output unit configured to output a composition that is determined to be the manufacturable material.

## Description

### Technical Field

The present invention relates to an apparatus, a method, and a program for a material search.

### Background Art

Conventionally, in many fields, material searches of searching for new and alternative materials are performed. For example, in development of alloys, various compositions of alloys (e.g., percentage by weight of each component) are used to prototype materials. Then, a method of searching for a material that meets required properties by measuring properties of a material that has been successfully prototyped is used.

In recent years, material searches based on computer analysis called material informatics are also used (Patent Document 1, Patent Document 2, and so on).

### [Prior Art Document]

### [Patent Document]

[Patent Document 1] Japanese Laid-open Patent Application Publication No. 2001-220638
[Patent Document 2] Japanese Laid-open Patent Application Publication No. 2004-149859

### Summary of the Invention

### Problem to be Solved by the Invention

However, at present, determining whether a material is manufacturable depends on human experience. Thus, even if a prototype is attempted, for some reasons (e.g., cavities formed at a casting stage), there may be cases where a material whose properties can be measured cannot be produced. Additionally, with material informatics, even if a material that meets required properties is found, there may be cases where the material cannot be actually produced.

Therefore, the present invention is intended to improve the efficiency of the material search.

### Means for Solving the Problem

The present invention has the following configuration.
[1] A material search apparatus including a composition obtaining unit configured to obtain a candidate composition, a thermodynamic calculation unit configured to perform thermodynamic calculation on the candidate composition, a manufacturing determining unit configured to determine whether the candidate composition is a manufacturable material based on a correspondence relation between a result of the thermodynamic calculation performed on a predetermined composition and manufacturability indicating whether a material having the predetermined composition is manufacturable, and an output unit configured to output a composition that is determined to be the manufacturable material
[2] The material search apparatus as described in claim [1], wherein the result of the thermodynamic calculation is phase fractions of a compound in an equilibrium state at each temperature
[3] The material search apparatus as described in [1] or [2], wherein the correspondence relation is defined by a manufacturability learned model or a set of rules, the manufacturability learned model being a model on which machine learning has been performed by using the result of the thermodynamic calculation performed on the predetermined composition and the manufacturability indicating whether the material having the predetermined composition is manufacturable as training data, and the set of rules being for deriving the manufacturability from the result of the thermodynamic calculation performed on the predetermined composition
[4] The material search apparatus as described in any one of [1] to [3], wherein the composition obtaining unit further obtains a candidate manufacturing condition, and the material search apparatus includes a property prediction unit configured to predict a property of a material having the candidate composition to be manufactured under the candidate manufacturing condition based on a correspondence relation between a predetermined composition and a predetermined manufacturing condition, and a property of a material having the predetermined composition that is manufactured under the predetermined manufacturing condition, a required property obtaining unit configured to obtain a required property for the material having the candidate composition to be manufactured under the candidate manufacturing condition, and a property determining unit configured to determine whether the predicted property meets the obtained required property
[5] The material search apparatus as described in [4], wherein the correspondence relation between the predetermined composition and the predetermined manufacturing condition, and the property of the material having the predetermined composition that is manufactured under the predetermined manufacturing condition is defined by a property prediction learned model or a set of rules based on statistical analysis, the property prediction learned model being a model on which machine learning has been performed by using the predetermined composition and the predetermined manufacturing condition, and the property of the material having the predetermined composition that is manufactured under the predetermined manufacturing condition, as training data
[6] The material search apparatus as described in [4], wherein, with respect to a composition used when the property determining unit determines that the predicted property meets the required property, the manufacturing determining unit determines whether a material having the composition is manufacturable
[7] The material search apparatus as described in [4], wherein, with respect to the composition that is determined by the manufacturing determining unit to be the manufacturable material, the property determining unit determines whether the predicted property meets the required property, and wherein the output unit outputs a composition that is determined to be the manufacturable material and determined to satisfy the required property
[8] The material search apparatus as described in [4], wherein determining whether the candidate composition is the manufacturable material and determining whether the predicted property meets the required property are performed in parallel, and wherein the output unit outputs a composition that is determined to be the manufacturable material and determined to satisfy the required property
[9] A method includes a step of obtaining a candidate composition, a step of performing thermodynamic calculation on the candidate composition, a step of determining whether the candidate composition is a manufacturable material based on a correspondence relation between a result of the thermodynamic calculation performed on a predetermined composition and manufacturability indicating whether a material having the predetermined composition is manufacturable, and a step of outputting a composition that is determined to be the manufacturable material
[10] A program for causing a computer to function as a composition obtaining unit configured to obtain a candidate composition, a thermodynamic calculation unit configured to perform thermodynamic calculation on the candidate composition, a manufacturing determining unit configured to determine whether the candidate composition is a manufacturable material based on a correspondence relation between a result of the thermodynamic calculation performed on a predetermined composition and manufacturability indicating whether a material having the predetermined composition is manufacturable, and an output unit configured to output a composition that is determined to be the manufacturable material

### Effect of the Invention

According to the present invention, the efficiency of the material search can be improved.

### Brief Description of the Drawings

FIG. 1 is a drawing illustrating the entirety of a system configuration including a material search apparatus according to one embodiment of the present invention;
FIG. 2 is a drawing illustrating the hardware configuration of the material search apparatus according to one embodiment of the present invention;
FIG. 3 is a drawing that describes each phase of a process of a material search according to one embodiment of the present invention;
FIG. 4 is a drawing that describes the manufacturability learning phase of the process of the material search according to one embodiment of the present invention;
FIG. 5 is a drawing that describes the property prediction learning phase of the process of the material search according to one embodiment of the present invention;
FIG. 6 is a drawing illustrating functional blocks of the material search apparatus according to one embodiment of the present invention;
FIG. 7 is a flowchart illustrating a flow of the screening process according to one embodiment of the present invention;
FIG. 8 is a flowchart illustrating a flow of the screening process according to one embodiment of the present invention;
FIG. 9 is another flowchart illustrating the flow of the screening process according to one embodiment of the present invention;
FIG. 10 is still another flowchart illustrating the flow of the screening process according to one embodiment of the present invention; and
FIG. 11 is a drawing that describes the effect of screening according to one embodiment of the present invention.

### Embodiment for Carrying Out the Invention

In the following, an embodiment will be described with reference to the accompanying drawings. In the present specification and the drawings, components having substantially the same functional configuration are referenced by the same reference signs, and the overlapping description is omitted.

Although, in the present specification, development of aluminum alloys is described as an example, the present invention may be applied to various alloy materials overall, such as Fe alloys, Cu alloys, Ni alloys, Co alloys, Ti alloys, Mg alloys, Mn alloys, Zn alloys, and the like. Further, the present invention may be applied to alloy materials of multiple compositions or to alloy materials manufactured by combinations of multiple manufacturing conditions and processes (i.e., temperature, pressure, processing, and the like).

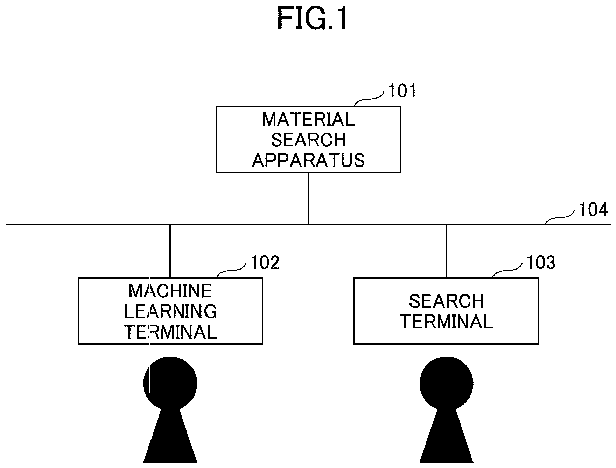

FIG. 1 is a drawing illustrating the entirety of a system configuration including a material search apparatus 101 according to one embodiment of the present invention. The system may include the material search apparatus 101, a machine learning terminal 102, and a search terminal 103. The material search apparatus 101 may transmit data to and receive data from the machine learning terminal 102 through a given network 104. The material search apparatus 101 may transmit data to and receive data from the search terminal 103 through the given network 104. Each of those will be described below.

The material search apparatus 101 is an apparatus for determining, based on a composition of a material, whether a material having the composition is manufacturable. The material search apparatus 101 is preferably an apparatus for determining, based on a composition and a manufacturing condition of a material, whether a property of a material having the composition that is manufactured under the manufacturing condition meets a property required for the material (which will be hereinafter referred to as the "required property ").

Specifically, the material search apparatus 101 determines whether a material having a composition specified by the search terminal 103 is manufacturable. The material search apparatus 101 may be configured to determine whether a property of a material manufactured according to a composition and manufacturing condition specified by the search terminal 103 meets a required property specified by the search terminal 103.

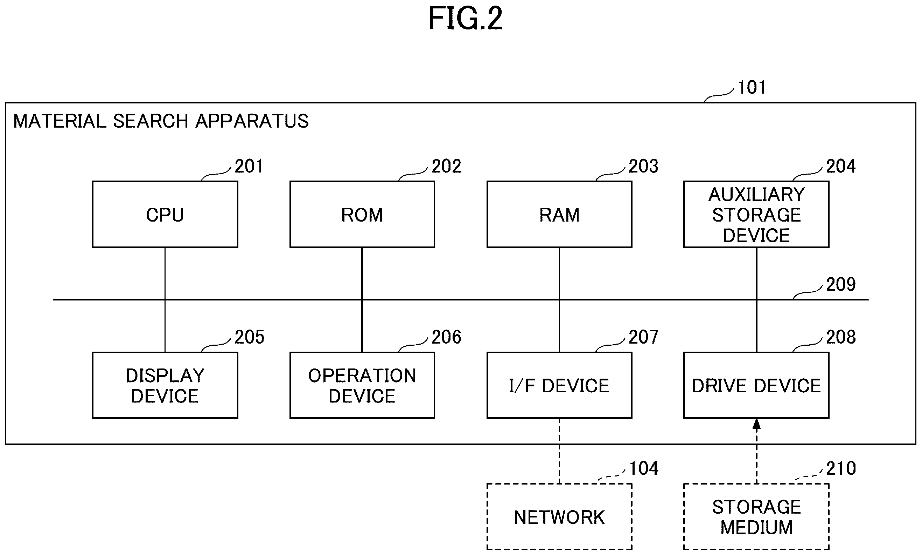

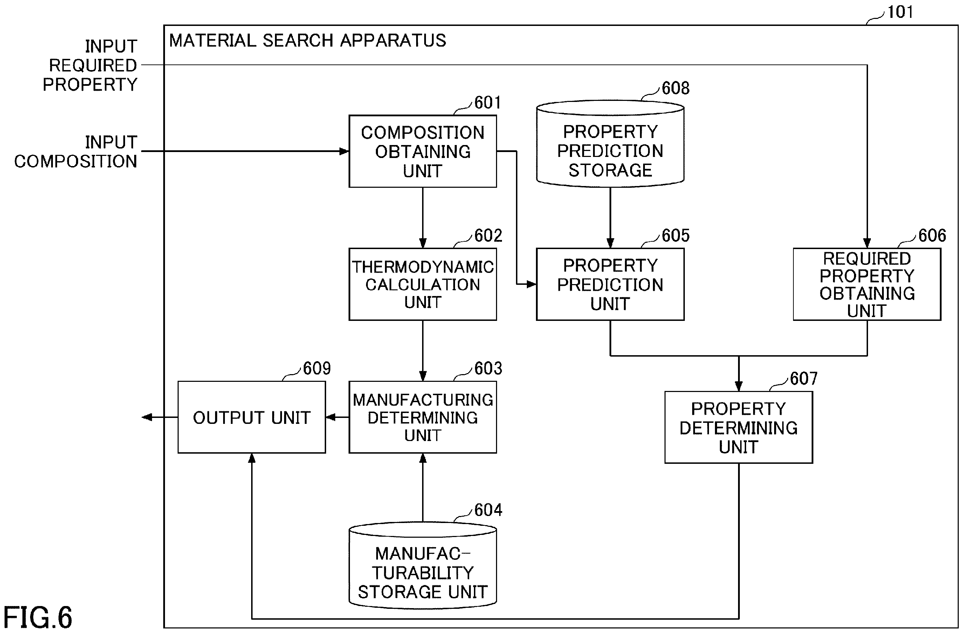

The material search apparatus 101 may be configured to perform machine learning based on data obtained from the machine learning terminal 102 to generate a learned model used to determine whether a material is manufacturable (which will be hereinafter also referred to as a manufacturability learned model). The material search apparatus 101 may be configured to perform machine learning based on data obtained from the machine learning terminal 102 to generate a learned model used to predict a property of a material (which will be hereinafter also referred to as a property prediction learned model). With reference to FIG. 2 and FIG. 6, the material search apparatus 101 will be described in detail later.

If the material search apparatus 101 uses the above-described manufacturability learned model, the machine learning terminal 102 is a terminal used by a person who inputs training data to generate the manufacturability learned model. The machine learning terminal 102 may be a terminal used by a person who inputs training data to generate the property prediction learned model. Specifically, the machine learning terminal 102 may transmit the training data input to the machine learning terminal 102 (which will be described in detail later) to the material search apparatus 101. The machine learning terminal 102 is, for example, a computer such as a personal computer.

The search terminal 103 is a terminal used by a person who wants to perform a material search. Specifically, the search terminal 103 may transmit, to the material search apparatus 101, data of a composition input to the search terminal 103. The search terminal 103 may transmit, to the material search apparatus 101, data of a manufacturing condition input to the search terminal 103. The search terminal 103 may transmit, to the material search apparatus 101, data of a required property input to the search terminal 103. The search terminal 103 may receive, from the material search apparatus 101, data of a composition determined to be a manufacturable material. The search terminal 103 may receive, from the material search apparatus 101, data of a composition determined to satisfy the required property. The search terminal 103 may receive, from the material search apparatus 101, data of a composition determined to be a manufacturable material and determined to satisfy the required property. The search terminal 103 is, for example, a computer such as a personal computer.

In the present specification, the machine learning terminal 102 and the search terminal 103 are described as separate computers, but the machine learning terminal 102 and the search terminal 103 may be implemented on a single computer. Additionally, the material search apparatus 101 may have some or all of functions of the machine learning terminal 102. The material search apparatus 101 may have some or all of functions of the search terminal 103.

### <Hardware configuration of the material search apparatus 101>

FIG. 2 is a drawing illustrating the hardware configuration of the material search apparatus 101 according to one embodiment of the present invention. The material search apparatus 101 includes a central processing unit (CPU) 201, a read only memory (ROM) 202, and a random access memory (RAM) 203. The CPU 201, the ROM 202, and the RAM 203 form what is called a computer.

The material search apparatus 101 further includes an auxiliary storage device 204, a display device 205, an operation device 206, an interface (I/F) device 207, and a drive device 208. Each hardware component of the material search apparatus 101 is interconnected with one another through a bus 209.

The CPU 201 is an arithmetic device that executes various programs installed in the auxiliary storage device 204.

The ROM 202 is a non-volatile memory. The ROM 202 functions as a main storage device that stores various programs, data, and the like that are necessary for the CPU 201 to execute various programs installed in the auxiliary storage device 204. Specifically, the ROM 202 functions as a main storage device that stores a boot program and the like, such as a basic input/output system (BIOS) and Extensible Firmware Interface (EFI).

The RAM 203 is a volatile memory, such as a dynamic random access memory (DRAM) or a static random access memory (SRAM). The RAM 203 functions as a main storage device that provides a workspace in which various programs installed in the auxiliary storage device 204 are deployed when various programs are executed by the CPU 201.

The auxiliary storage device 204 is an auxiliary storage device that stores various programs and information used when various programs are executed.

The display device 205 is a display device that displays an internal state or the like of the material search apparatus 101.

The operation device 206 is an input device used by an administrator of the material search apparatus 101 to input various instructions to the material search apparatus 101.

The I/F device 207 is a communication device that connects to the network 104 and communicates with the machine learning terminal 102 and the search terminal 103.

The drive device 208 is a device that sets a storage medium 210. The storage medium 210 herein includes a medium that optically, electrically, or magnetically records information, such as a CD-ROM, a flexible disk, a magnetooptical disk, or the like. Additionally, the storage medium 210 may include a semiconductor memory or the like that electrically records information, such as an erasable programmable read only memory (EPROM), a flash memory, or the like.

Various programs to be installed in the auxiliary storage device 204 are installed, for example, when the distributed storage medium 210 is set in the drive device 208 and various programs recorded in the storage medium 210 are read by the drive device 208. Alternatively, various programs to be installed in the auxiliary storage device 204 may be installed by being downloaded from another network different from the network 104 through the I/F device 207.

### <Overview of a process of the material search>

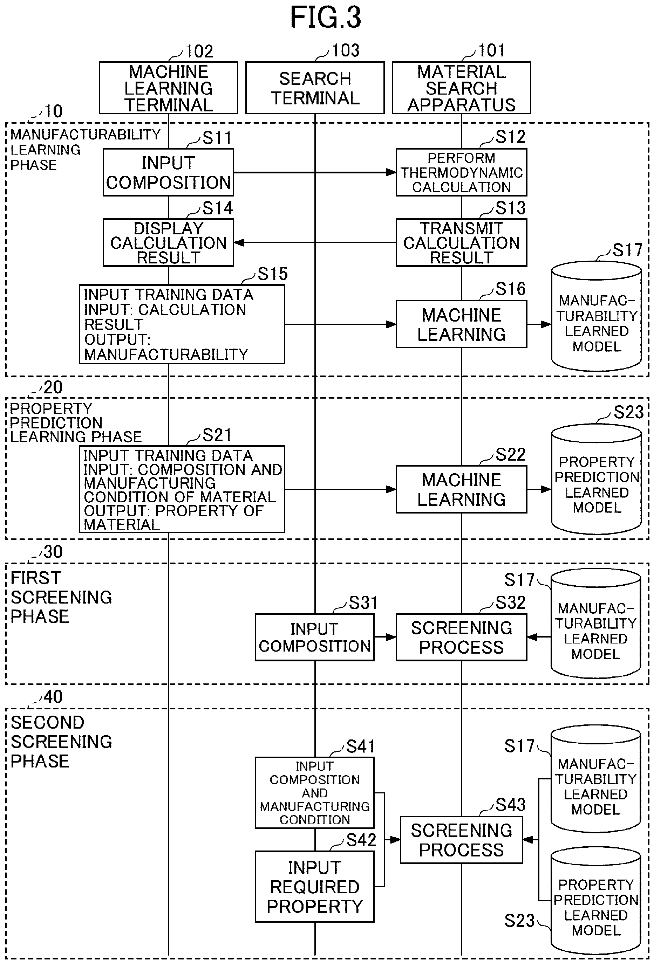

FIG. 3 is a drawing that describes each phase of a process of the material search according to one embodiment of the present invention. As illustrated in FIG. 3, the process of the material search apparatus 101 can be divided into four phases. Specifically, the process of the material search apparatus 101 can be divided into phases of "a manufacturability learning phase 10", "a property prediction learning phase 20", "a first screening phase 30", and "a second screening phase 40". The order of the phases may be changed as long as there is no conflict with respect to the order of preparation and processing of the phases. If only manufacturability is to be determined, the first screening phase is performed, and if both manufacturability and property prediction are to be determined, the second screening phase is performed. Each phase will be described below.

### <Manufacturability learning phase>

The manufacturability learning phase will be described.
- In step 11 (S11), the machine learning terminal 102 may transmit data of a composition of a material input to the machine learning terminal 102 to the material search apparatus 101. The composition of the aluminum alloy material is, for example, a percentage by weight (wt%) of each component (e.g., element species such as Si, Fe, Cu, Mn, Mg, Cr, Ni, Zn, Ti, Na, V, Pb, Sn, B, Bi, Zr, O). The percentage by weight (wt%) of the aluminum is expressed as 100 - (the sum of the percentages by weight of the above-described elements).
- In step 12 (S12), the material search apparatus 101 may perform thermodynamic calculation on the composition obtained in S11. The material search apparatus 101 may perform the thermodynamic calculation, for example, by using a calculation of phase diagrams (CALPHAD) method. In the CALPHAD method, an equilibrium state reached at each temperature can be calculated for the alloy of the composition, and a result of the thermodynamic calculation is, for example, phase fractions of a compound included in the equilibrium state at each temperature.

In the thermodynamic calculation using the CALPHAD method described above, the Scheil-Gulliver (SG) model may be used to perform thermodynamic calculations. In the SG model approximation expression, no diffusion in a solid phase and uniform composition in a liquid phase is assumed. By using the SG model, if the alloy material of the composition is cooled from a high-temperature liquid phase, a liquid phase composition present at each temperature over a solidification process and the type and amount of a solid phase component solidified by precipitation can be calculated. A result of the thermodynamic calculation is, for example, phase fractions of a compound included at each temperature that reflect the solid phase component precipitated during the solidification process.

Data of the composition and manufacturing condition of the material may be input to the machine learning terminal 102. In this case, simulation using a phase field method may be performed in conjunction with the thermodynamic calculation using the CALPHAD method. The manufacturing conditions of the material include, for example, the cooling speed, thermal refining (processing pattern in manufacturing), the temperature and retention time of each process (annealing, solution treatment, artificial aging, natural aging, hot working, cold working, and stabilization) and processing conditions (rate of work, push ratio, reduction of area, a product shape, and the like) in casting. In the phase field process, for example, phase transformation and growth of a crystal grain can be calculated, reflecting the temperature and time of the manufacturing condition for the alloy material of the composition. The result of the thermodynamic calculation is, for example, phase fractions of a compound included after a process of the phase transformation and the number average particle size of a compound.
- In step 13 (S13), the material search apparatus 101 may transmit the data of the result of the thermodynamic calculation performed in S12 to the machine learning terminal 102. The result of the thermodynamic calculation is, for example, phase fractions of a compound included in the equilibrium state at each temperature.
- In step 14 (S14), the machine learning terminal 102 may display the result of the thermodynamic calculation obtained in S13 on a display device of the machine learning terminal 102.
- In step 15 (S15), the machine learning terminal 102 may transmit, to the material search apparatus 101, training data input to the machine learning terminal 102. With respect to the training data, input data is the result of the thermodynamic calculation, and output data is manufacturability. The manufacturability may, for example, indicate whether manufacturable, or may indicate a probability of being manufacturable.
- In step 16 (S16), the material search apparatus 101 may perform machine learning by using data obtained in S15 as the training data.

Thus, machine learning is performed by using the result of the thermodynamic calculation and the manufacturability as the training data to generate a manufacturability learned model S17.

If data of the composition and manufacturing condition of the material is input to the machine learning terminal 102, with respect to the training data, input data is the result of the thermodynamic calculation and the manufacturing condition and output data is the manufacturability. In this case, machine learning is performed by using the result of the thermodynamic calculation and the manufacturing condition, and the manufacturability as the training data to generate the manufacturability learned model S17. It is selected whether the data of the composition of the material or the data of the composition and manufacturing condition of the material is input as the data input to the machine learning terminal 102, in accordance with data input to the search terminal 103 during the first screening phase or the second screening phase, which will be described below.

### <Property prediction learning phase>

The property prediction learning phase will be described.
- In step 21 (S21), the machine learning terminal 102 may transmit, to the material search apparatus 101, training data input to the machine learning terminal 102. With respect to the training data, input data is the composition and manufacturing condition of the material and output data is a property of the material. The composition of the material is substantially the same as the above-described composition used in the manufacturability learning phase. The manufacturing conditions of the material include, for example, the cooling speed, thermal refining (processing pattern in manufacturing), the temperature and retention time of each process (annealing, solution treatment, artificial aging, natural aging, hot working, cold working, and stabilization) and processing conditions (rate of work, push ratio, reduction of area, a product shape, and the like) in casting. The properties of the material include, for example, tensile strength, 0.2% proof stress, stretch, coefficients of linear expansion, Young's modulus, Poisson's ratio, fatigue properties, hardness, creep strength, creep strain, shear strength, specific heat, thermal conductivity, electrical resistivity, density, a solidus line, a liquidus line, and the like.
- In step 22 (S22), the material search apparatus 101 may perform machine learning by using the data obtained in S21 as the training data.

As described above, machine learning is performed by using the composition and manufacturing condition of the material and the property of the material as the training data to generate a property prediction learned model S23.

As the training data, the input data may be the composition of the material and the output data may be the property of the material. In this case, machine learning is performed by using the composition of the material and the property of the material as the training data to generate the property prediction learned model S23. Whether the data of the composition of the material, or the data of the composition of the material and manufacturing condition, is input as the data input to the machine learning terminal 102, is selected in accordance with the data input to the search terminal 103 in the first screening phase or the second screening phase, which will be described below.

### <First screening phase>

The first screening phase will be described. In the first screening phase, a composition determined to be a manufacturable material is screened.
- In step 31 (S31), the search terminal 103 may transmit, to the material search apparatus 101, the data of the composition of the material that is input to the search terminal 103. In accordance with the composition, it is determined whether the material is manufacturable.
- In step 32 (S32), the material search apparatus 101 may determine whether the material is manufacturable in accordance with the composition obtained in S31 to screen the composition determined to be a manufacturable material. Here, the material search apparatus 101 may determine whether the material is manufacturable in accordance with the composition obtained in S31 by using the manufacturability learned model S17.

To the machine learning terminal 102, the data of the composition and manufacturing condition of the material may be input.

### <Second screening phase>

The second screening phase will be described. In the second screening phase, a composition determined to be a manufacturable material and determined to satisfy the required property is screened.
- In step 41 (S41), the search terminal 103 may transmit, to the material search apparatus 101, the data of the composition and manufacturing condition of the material that is input to the search terminal 103. Whether a property of the material having the composition that is manufactured under the manufacturing condition meets a required property, is determined.
- In step 42 (S42), the search terminal 103 may transmit, to the material search apparatus 101, data of the required property that is input to the search terminal 103. The required property is a property required for the material. Here, S41 and S42 may be performed simultaneously or S41 may be performed after S42.
- In step 43 (S43), the material search apparatus 101 may determine whether a property of the material having the composition obtained in S41 that is manufactured under the manufacturing condition obtained in S41 meets the required property obtained in S42. Similarly with the first screening phase 30, the material search apparatus 101 may determine whether the material having the composition obtained in S41 is manufacturable. The material search apparatus 101 can then screen a composition determined to be a manufacturable material and determined to satisfy the required property. Here, the material search apparatus 101 may predict the property of the material to be manufactured having the composition obtained in S41 by using the property prediction learned model S23.

The data input to the search terminal 103 may be the composition of material.

As described above, determination may be performed as to whether the material is manufacturable based on the composition of the material, or based on the composition and manufacturing condition of the material. Additionally, determination may be performed as to whether the required property is met based on the composition and manufacturing condition of the material, or based on the composition of the material.

### <Specific example of the manufacturability learning phase>

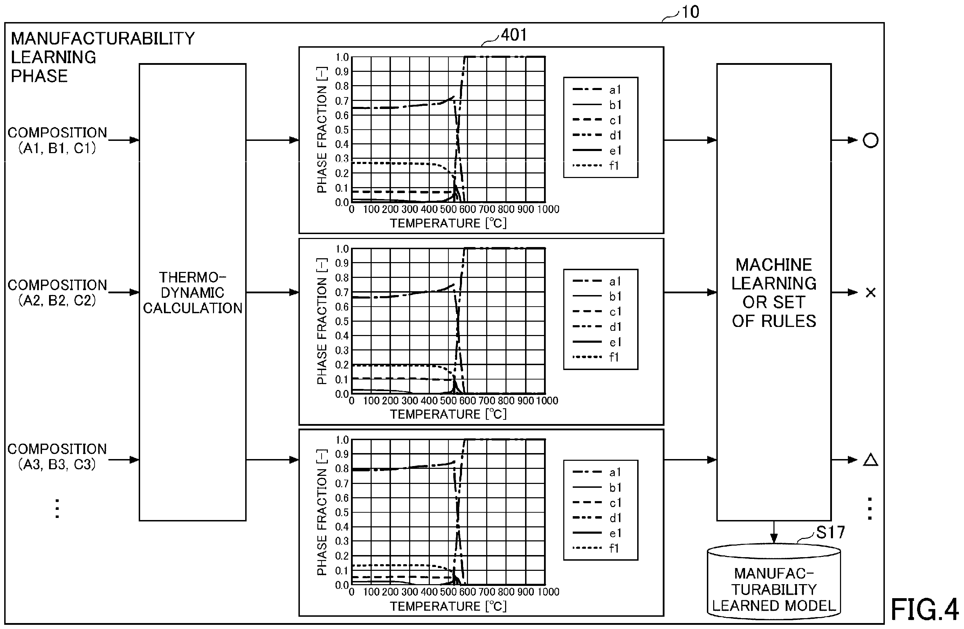

FIG. 4 is a drawing that describes the manufacturability learning phase 10 of the process of the material search according to one embodiment of the present invention. In the manufacturability learning phase 10, a correspondence relation, between a result of the thermodynamic calculation for a predetermined composition and the manufacturability indicating whether the material having the predetermined composition is manufacturable, is generated. First, the material search apparatus 101 performs thermodynamic calculation on each composition (e.g., a composition (A1, B1, C1), a composition (A2, B2, C2), a composition (A3, B3, C3), and the like) . A result 401 of the thermodynamic calculation is, for example, phase fractions of a compound in an equilibrium state at each temperature as illustrated in FIG. 4. Next, the material search apparatus 101 performs machine learning by using the training data in which input data is the result of the thermodynamic calculation and output data is the manufacturability (e.g., o, ×, Δ, ...), to generate the manufacturability learned model S17. Alternatively, the material search apparatus 101 may use a set of rules (e.g., a rule based on knowledge from experience of a skilled technician such that if a solid-liquid coexistence temperature range between a liquidus line and a solidus line in the state diagram reaches 40°C or higher, casting cracks occur and a material cannot be manufactured), instead of machine learning. That is, the material search apparatus 101 may determine the manufacturability based on the manufacturability learned model S17 generated by machine learning, or the material search apparatus 101 may determine the manufacturability based on the set of rules.

### <Specific example of the property prediction learning phase>

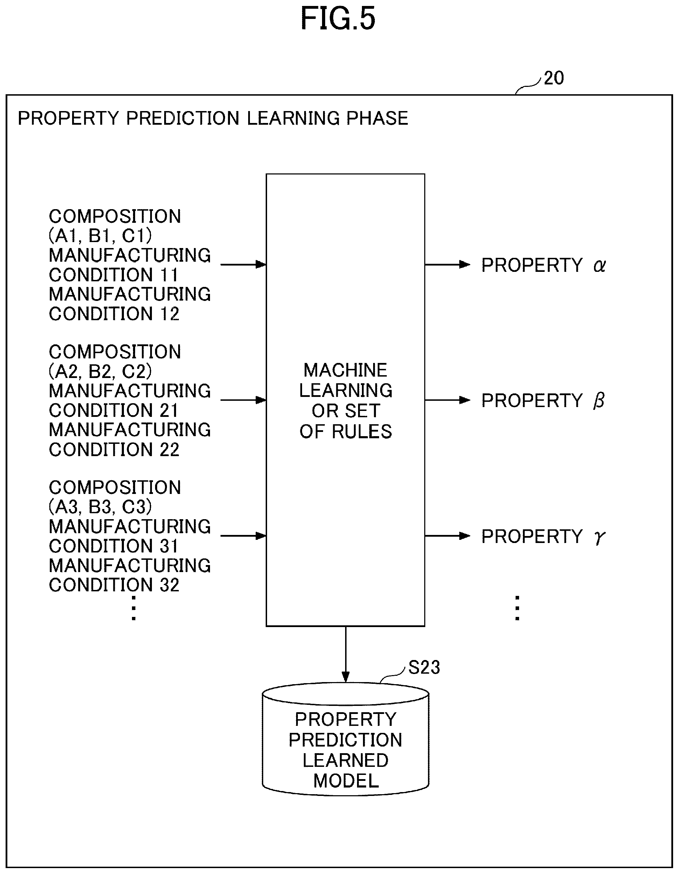

FIG. 5 is a drawing that describes the property prediction learning phase 20 of a process of the material search according to one embodiment of the present invention. In the property prediction learning phase 20, a correspondence relation, between a predetermined composition and manufacturing condition and the property of the material having the predetermined composition that is manufactured under the manufacturing condition, is generated. The material search apparatus 101 performs machine learning by using training data in which input data is compositions (e.g., a composition (A1, B1, C1), a composition (A2, B2, C2), a composition (A3, B3, C3), ...) and manufacturing conditions (e.g., (a manufacturing condition 11 and a manufacturing condition 12), (a manufacturing condition 21 and a manufacturing condition 22), (a manufacturing condition 31 and a manufacturing condition 32), ...) and output data is properties (e.g., a property α, a property β, a property γ, ...) to generate a property prediction learned model S23. Alternatively, the material search apparatus 101 may use a set of rules (e.g., a rule based on statistical analysis) instead of machine learning. That is, the material search apparatus 101 may predict the property based on the property prediction learned model S23 generated by machine learning or predict the property based on the set of rules.

### <Functional blocks of the material search apparatus 101>

FIG. 6 is a drawing illustrating functional blocks of the material search apparatus 101 according to one embodiment of the present invention. The material search apparatus 101 includes a composition obtaining unit 601, a thermodynamic calculation unit 602, a manufacturing determining unit 603, a manufacturability storage unit 604, and an output unit 609. The material search apparatus 101 may further include a property prediction unit 605, a required property obtaining unit 606, a property determining unit 607, and a property prediction storage unit 608. The material search apparatus 101 functions as the composition obtaining unit 601, the thermodynamic calculation unit 602, the manufacturing determining unit 603, and the output unit 609 by executing a program. The material search apparatus 101 may further function as the property prediction unit 605, the required property obtaining unit 606, and the property determining unit 607. Each of them will be described below.

The composition obtaining unit 601 obtains an input composition of the material. The composition obtaining unit 601 may obtain a candidate manufacturing condition of the material. Specifically, the composition obtaining unit 601 receives the data of the composition input to the search terminal 103 from the search terminal 103. As described above, the composition of the aluminum alloy material is, for example, the percentage by weight (wt%) of each component (i.e., elemental species such as Si, Fe, Cu, Mn, Mg, Cr, Ni, Zn, Ti, Na, V, Pb, Sn, B, Bi, Zr, and O). The percentage by weight (wt%) of the aluminum is expressed by 100 - (the sum of the percentages by weight of the above-described elements). The composition obtaining unit 601 may receive the data of the manufacturing condition input to the search terminal 103 from the search terminal 103. As described above, the manufacturing conditions of the material include, for example, the cooling speed, thermal refining (processing pattern in manufacturing), the temperature and retention time of each process (annealing, solution treatment, artificial aging, natural aging, hot working, cold working, and stabilization) and processing conditions (rate of work, push ratio, reduction of area, a product shape, and the like) in casting. Here, values of the composition of the material (e.g., the percentages by weight) may be specified as ranges such as maximum values and minimum values or specified as specific values. If the values of the composition of the material are specified as ranges such as maximum values and minimum values, the determination may be performed on a composition randomly generated within the specified ranges, or the determination may be performed on a composition at a grid point in the specified ranges. The composition obtaining unit 601 may store the obtained data in a memory so that the thermodynamic calculation unit 602 can refer to the obtained data.

The thermodynamic calculation unit 602 performs the thermodynamic calculation on the composition obtained by the composition obtaining unit 601. Specifically, the thermodynamic calculation unit 602 may perform the thermodynamic calculation by, for example, using a calculation of phase diagrams (CALPHAD) method. A result of the thermodynamic calculation is, for example, phase fractions of a compound in the equilibrium state at each temperature. In the CALPHAD method, an equilibrium state reached at each temperature can be calculated for the alloy of the composition, and a result of the thermodynamic calculation is, for example, phase fractions of a compound included in the equilibrium state at each temperature.

In the thermodynamic calculation using the CALPHAD method described above, the Scheil-Gulliver (SG) model may be used to perform the thermodynamic calculation. In the SG model approximation expression, no diffusion in the solid phase and uniform composition in the liquid phase are assumed. By using the SG model, if the alloy material of the composition is cooled from a high-temperature liquid phase, a liquid phase composition present at each temperature over a solidification process and the type and amount of a solid phase component solidified by precipitation can be calculated. A result of the thermodynamic calculation is, for example, phase fractions of a compound included at each temperature that reflect the solid phase component precipitated during the solidification process.

If the composition obtaining unit 601 obtains the data of the composition and manufacturing condition of the material, a simulation using the phase field method may be further performed in conjunction with the thermodynamic calculation using the CALPHAD method. In the phase field method, for example, phase transformation and growth of a crystal grain can be calculated, reflecting the temperature and time of the manufacturing condition for the alloy material of the composition. The result of the thermodynamic calculation is, for example, phase fractions of a compound included after a process of the phase transformation and the number average particle size of a compound.

The thermodynamic calculation unit 602 may store the result of the thermodynamic calculation in memory so that the manufacturing determining unit 603 can refer to the result.

The manufacturing determining unit 603 determines whether a material having the composition obtained by the composition obtaining unit 601 is manufacturable. Specifically, the manufacturing determining unit 603 determines whether a material having the composition obtained by the composition obtaining unit 601 is manufacturable based on "the correspondence relation between the result of the thermodynamic calculation for a predetermined composition and the manufacturability indicating whether the material having the predetermined composition is manufacturable" stored in the manufacturability storage unit 604 and the result of the thermodynamic calculation performed by the thermodynamic calculation unit 602. Here, the manufacturing determining unit 603 may determine the probability of being manufacturable (e.g., a level indicating whether the manufacturability is high or low). The manufacturing determining unit 603 may store the composition of the material determined to be manufacturable in a memory so that the output unit 609 can refer to the determined composition of the material.

The manufacturability storage unit 604 may store "the correspondence relation between the result of the thermodynamic calculation for a predetermined composition and the manufacturability indicating whether the material having the predetermined composition is manufacturable". Specifically, the manufacturability storage unit 604 may store the manufacturability learned model S17 described with reference to FIG. 3 and FIG. 4. Additionally, the manufacturability storage unit 604 may store the set of rules described with reference to FIG. 4.

As described above, in one embodiment of the present invention, whether a material having the composition is manufacturable is determined by using the correspondence relationship between information that is calculated from the composition and that is necessary to determine whether manufacturable (e.g., the result of the thermodynamic calculation for the composition) and the manufacturability, rather than the composition itself.

The property prediction unit 605 may predict a property of the material having the composition obtained by the composition obtaining unit 601 that is manufactured under the manufacturing condition obtained by the composition obtaining unit 601. Specifically, the property prediction unit 605 may predict the property of the material having the composition obtained by the composition obtaining unit 601 that is manufactured under the manufacturing condition obtained by the composition obtaining unit 601 based on "the correspondence relation between a predetermined composition and manufacturing condition and the property of the material having the predetermined composition that is manufactured under the manufacturing condition" stored in the property prediction storage unit 608 and the composition and manufacturing condition obtained by the composition obtaining unit 601. As described above, the properties of the material include, for example, tensile strength, 0.2% proof stress, stretch, coefficients of linear expansion, Young's modulus, Poisson's ratio, fatigue properties, hardness, creep strength, creep strain, shear strength, specific heat, thermal conductivity, electrical resistivity, density, a solidus line, a liquidus line, and the like. The property prediction unit 605 may store the predicted property in a memory so that the property determining unit 607 can refer to the predicted property.

The property prediction storage unit 608 may store "the correspondence relation between a predetermined composition and manufacturing condition and the property of the material having the predetermined composition that is manufactured under the manufacturing condition". Specifically, the property prediction storage unit 608 may store the property prediction learned model S23 described with reference to FIG. 3 and FIG. 5. Additionally, the property prediction storage unit 608 may store the set of rules described with reference to FIG. 5.

The required property obtaining unit 606 may obtain the required property. Specifically, the required property obtaining unit 606 may receive, from the search terminal 103, data of the property required for the material having the composition obtained by the composition obtaining unit 601 that is manufactured under the manufacturing condition obtained by the composition obtaining unit 601. As described above, the properties of the material include, for example, tensile strength, 0.2% proof stress, stretch, coefficients of linear expansion, Young's modulus, Poisson's ratio, fatigue properties, hardness, creep strength, creep strain, shear strength, specific heat, thermal conductivity, electrical resistivity, density, a solidus line, a liquidus line, and the like. The required property obtaining unit 606 may store the obtained data in a memory so that the property determining unit 607 can refer to the obtained data.

The property determining unit 607 may determine whether the property predicted by the property prediction unit 605 meets the required property obtained by the required property obtaining unit 606. Specifically, the property determining unit 607 may determine whether the property predicted by the property prediction unit 605 meets some or all of the required properties. The property determining unit 607 may store the composition of the material determined to meet the required property in a memory so that the output unit 609 can refer to the composition.

The output unit 609 outputs the composition determined by the manufacturing determining unit 603 to be a manufacturable material. The output unit 609 may output the composition determined by the property determining unit 607 to satisfy the required property. The output unit 609 may output the composition determined by the manufacturing determining unit 603 to be a manufacturable material and determined by the property determining unit 607 to satisfy the required property. Specifically, the output unit 609 may transmit data of one or more compositions to the search terminal 103.

### <Details of a screening process>

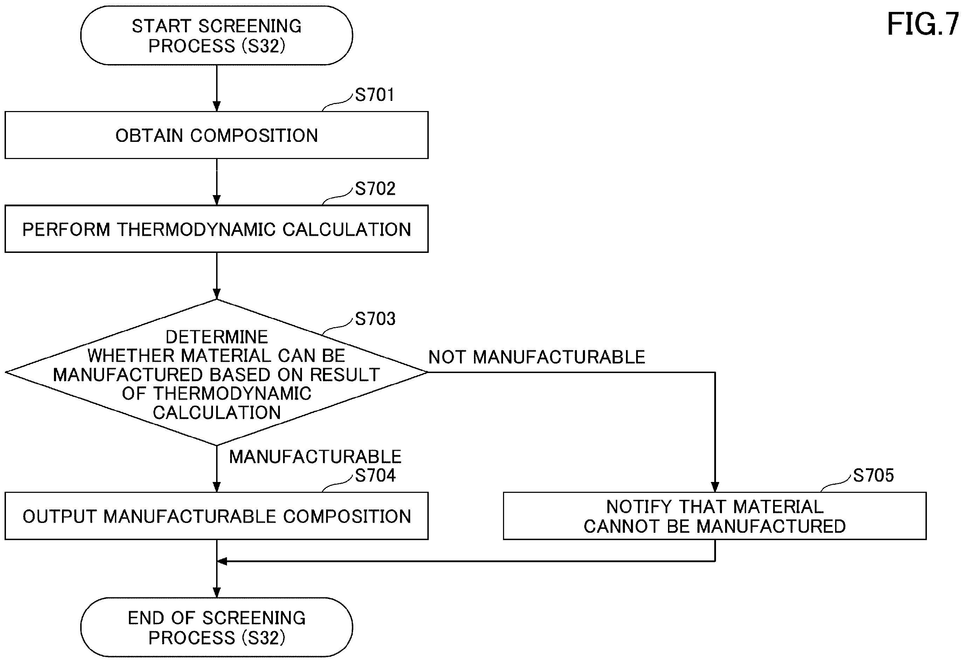

FIG. 7 is a flowchart illustrating a screening process flow according to one embodiment of the present invention. Specifically, FIG. 7 illustrates a flow of the screening process of S32 of FIG. 3 (i.e., a manufacturability determining process).

In step 701 (S701), the composition obtaining unit 601 obtains a candidate composition of the material.

In step 702 (S702), the thermodynamic calculation unit 602 performs the thermodynamic calculation on the composition obtained in S701.

In step 703 (S703), the manufacturing determining unit 603 determines whether a material having the composition obtained in S701 is manufacturable based on a result of the thermodynamic calculation performed in S702. If the material is manufacturable, the process proceeds to step 704. If the material is not manufacturable, the process proceeds to step 705. In step 705 (S705), the manufacturing determining unit 603 notifies the search terminal 103 that a material having the composition obtained in S701 is not manufacturable.

In step 704 (S704), the output unit 609 outputs the composition determined in S703 to be a manufacturable material.

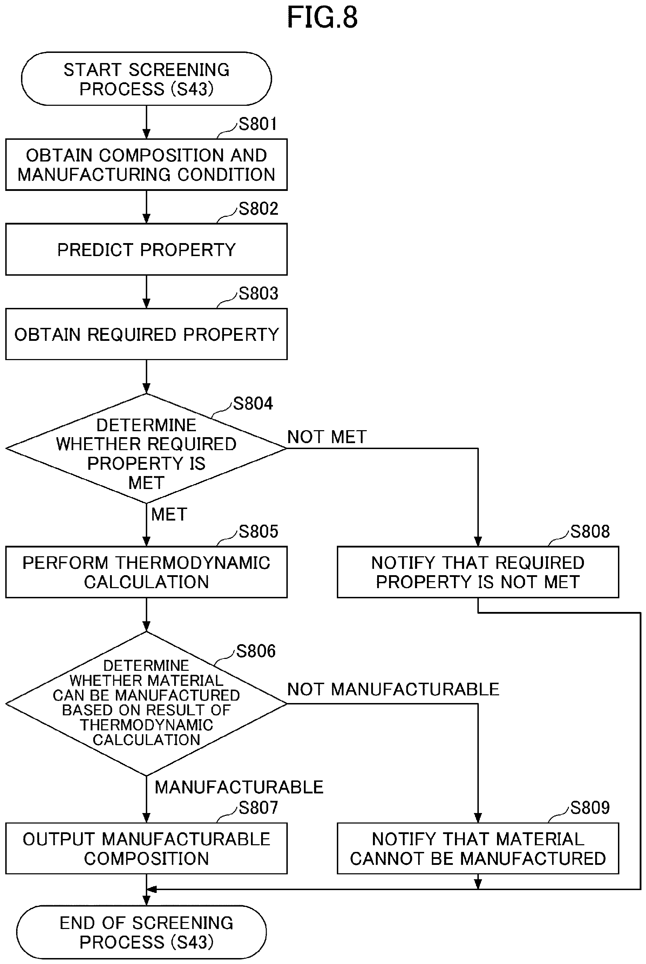

FIG. 8 is a flowchart illustrating a flow of the screening process according to one embodiment of the present invention. Specifically, FIG. 8 illustrates the flow of the screening process of S43 in FIG. 3 (i.e., the manufacturability determining process and a property determining process).

In step 801 (S801), the composition obtaining unit 601 obtains a candidate composition and a candidate manufacturing condition of the material.

In step 802 (S802), the property prediction unit 605 predicts a property of the material having the composition obtained in S801 that is manufactured under the manufacturing condition obtained in S801.

In step 803 (S803), the required property obtaining unit 606 obtains a required property. Here, S803 may be performed at any time as long as S803 is performed before S804, such as at the same time as S801.

In step 804 (S804), the property determining unit 607 determines whether the property predicted by S802 meets the required property obtained by S803. If the required property is met, the process proceeds to step 805. If the required property is not met, the process proceeds to step 808. In step 808 (S808), the property determining unit 607 notifies the search terminal 103 that the composition obtained in S801 does not meet the required property.

In step 805 (S805), the thermodynamic calculation unit 602 performs the thermodynamic calculation on the composition determined to meet the required property in S804.

In step 806 (S806), the manufacturing determining unit 603 determines whether a material having the composition obtained in S801 is manufacturable based on a result of the thermodynamic calculation performed in S805. If the material is manufacturable, the process proceeds to step 807. If the material is not manufacturable, the process proceeds to step 809. In step 809 (S809), the manufacturing determining unit 603 notifies the search terminal 103 that a material having the composition obtained in S801 is not manufacturable.

In step 807 (S807), the output unit 609 outputs the composition determined in S806 to be a manufacturable material.

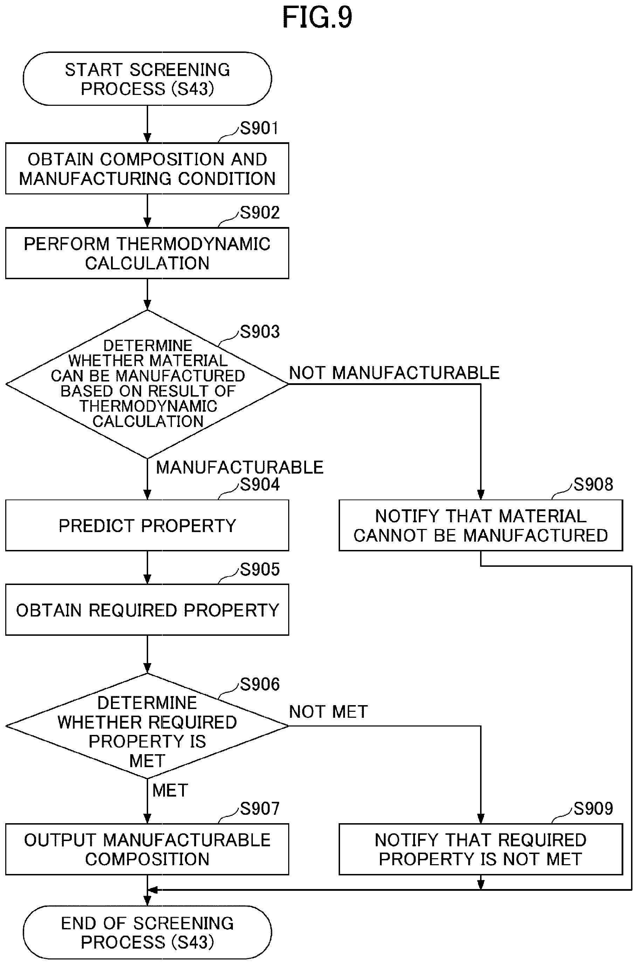

FIG. 9 is another flowchart illustrating a flow of the screening process according to one embodiment of the present invention. Specifically, FIG. 9 illustrates the flow of the screening process of S43 in FIG. 3 (i.e., the manufacturability determining process and the property determining process).

In step 901 (S901), the composition obtaining unit 601 obtains a candidate composition and a candidate manufacturing condition of the material.

In step 902 (S902), the thermodynamic calculation unit 602 performs the thermodynamic calculation on the composition obtained in S901.

In step 903 (S903), the manufacturing determining unit 603 determines whether a material having the composition obtained in S901 is manufacturable based on the result of the thermodynamic calculation performed in S902. If the material is manufacturable, the process proceeds to step 904. If the material is not manufacturable, the process proceeds to step 908. In step 908 (S908), the manufacturing determining unit 603 notifies the search terminal 103 that the material having the composition obtained in S901 is not manufacturable.

In step 904 (S904), the property prediction unit 605 predicts a property of the material having the composition obtained in S901 that is manufactured under the manufacturing condition obtained in S901.

In step 905 (S905), the required property obtaining unit 606 obtains a required property. Here, S905 may be performed at any time as long as S905 is performed before S906, such as at the same time as S901.

In step 906 (S906), the property determining unit 607 determines whether the property predicted by S904 meets the required property obtained in S905. If the required property is met, the process proceeds to step 907. If the required property is not met, the process proceeds to step 909. In step 909 (S909), the property determining unit 607 notifies the search terminal 103 that the composition obtained in S901 does not meet the required property.

In step 907 (S907), the output unit 609 outputs the composition that satisfies the required property as determined in S906.

In the processes of FIG. 8 and FIG. 9, the determined result may be notified in the middle of the processes. Specifically, the property determining unit 607 may output the composition determined to meet the required property in S804 of FIG. 8. The manufacturing determining unit 603 may output the composition determined in S903 of FIG. 9 to be a manufacturable material.

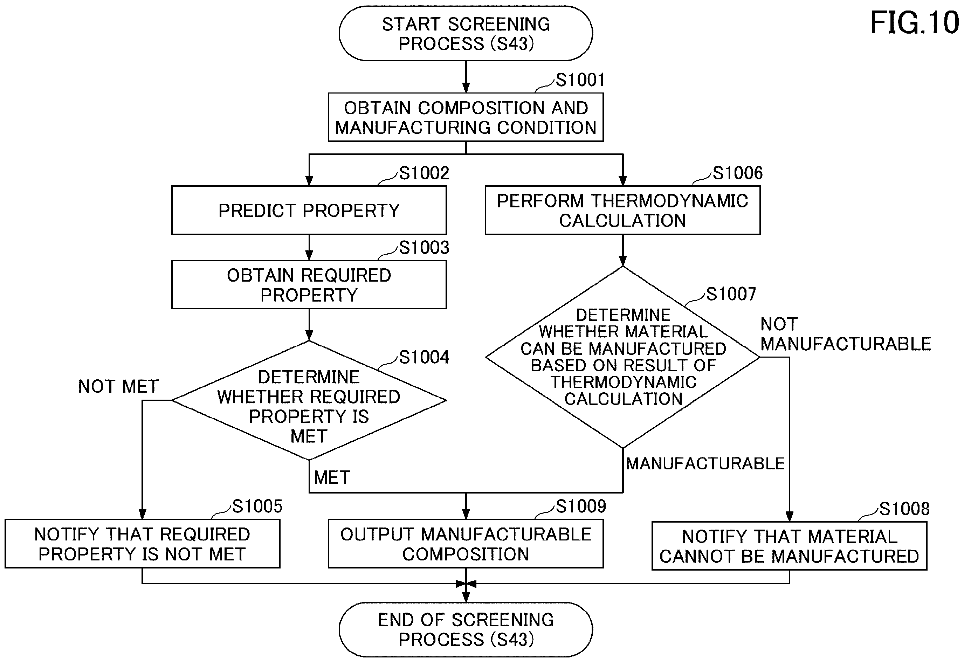

FIG. 10 is another flowchart illustrating a flow of the screening process according to one embodiment of the present invention. Specifically, FIG. 10 illustrates the flow of the screening process of S43 in FIG. 3 (i.e., the manufacturability determining process and the property determining process).

In step 1001 (S1001), the composition obtaining unit 601 obtains a candidate composition and a candidate manufacturing condition of the material. Subsequently, a process of step 1004 for determination and a process of step 1007 for determination are performed in parallel.

In step 1002 (S1002), the property prediction unit 605 predicts a property of the material having the composition obtained in S1001 that is manufactured under the manufacturing condition obtained in S1001.

In step 1003 (S1003), the required property obtaining unit 606 obtains a required property. Here, S1003 may be performed at any time as long as S1003 is performed before S1004, such as at the same time as S1001.

In step 1004 (S1004), the property determining unit 607 determines whether the property predicted in S1002 meets the required property obtained in S1003. If the required property is met, the process proceeds to step 1009. If the required property is not met, the process proceeds to step 1005. In step 1005 (S1005), the property determining unit 607 notifies the search terminal 103 that the composition obtained in S1001 does not satisfy the required property.

In step 1006 (S1006), the thermodynamic calculation unit 602 performs the thermodynamic calculation on the composition obtained in S1001.

In step 1007 (S1007), the manufacturing determining unit 603 determines whether a material having the composition obtained in S1001 is manufacturable based on the result of the thermodynamic calculation performed in S1006. If the material is manufacturable, the process proceeds to step 1009. If the material is not manufacturable, the process proceeds to step 1008. In step 1008 (S1008), the manufacturing determining unit 603 notifies the search terminal 103 that a material having the composition obtained in S1001 is not manufacturable.

In step 1009 (S1009), the output unit 609 outputs the composition that satisfies the required property as determined in S1004, and that is determined in S1007 to be a manufacturable material.

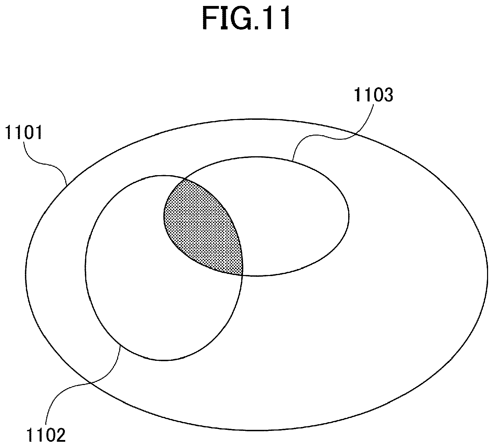

FIG. 11 is a drawing that describes the effect of the screening according to one embodiment of the present invention. As illustrated in FIG. 11, in one embodiment of the present invention, from candidates 1101 of the composition (i.e., a group of the candidate compositions of the material that is specified by the search terminal 103), a composition 1102 that is a manufacturable material can be screened. In one embodiment of the invention, from the candidates 1101 of the composition, a composition 1103 that satisfies the required property can be screened. In one embodiment of the invention, from the candidates 1101 of the composition, a composition that is a manufacturable material and that satisfies the required property (i.e., a portion having the dots in FIG. 11 that is included in both 1102 and 1103) can be screened.

As described above, in one embodiment of the present invention, it can be determined whether a material is manufacturable in advance, thereby reducing wasteful prototypes of the material.

It should be noted that the present invention is not limited to the above-described configurations, such as the configurations described in the above-described embodiments, and combinations with other elements. In these respects, changes can be made within the scope of the invention without departing from the spirit of the invention, and the configuration can be appropriately determined in accordance with the application form.

The present international application is based on and claims priority to Japanese Patent Application No. 2018-206018, filed on October 31, 2018, the entire contents of which are hereby incorporated herein by reference.

### Description of the Reference Numerals

- 101: material search apparatus
- 102: machine learning terminal
- 103: search terminal
- 104: network
- 10: manufacturability learning phase
- 20: property prediction learning phase
- 30: first screening phase
- 40: second screening phase
- s17: manufacturability learned model
- s23: property prediction learned model
- 401: calculation result
- 601: composition obtaining unit
- 602: thermodynamic calculation unit
- 603: manufacturing determining unit
- 604: manufacturability storage unit
- 605: property prediction unit
- 606: required property obtaining unit
- 607: property determining unit
- 608: property prediction storage
- 609: output unit

## Claims

1. A material search apparatus, comprising:
a composition obtaining unit configured to obtain a candidate composition;
a thermodynamic calculation unit configured to perform thermodynamic calculation on the candidate composition;
a manufacturing determining unit configured to determine whether the candidate composition is a manufacturable material based on a correspondence relation between a result of the thermodynamic calculation performed on a predetermined composition and manufacturability indicating whether a material having the predetermined composition is manufacturable; and
an output unit configured to output a composition that is determined to be the manufacturable material.

2. The material search apparatus as claimed in claim 1, wherein the result of the thermodynamic calculation is phase fractions of a compound in an equilibrium state at each temperature.

3. The material search apparatus as claimed in claim 1 or 2, wherein the correspondence relation is defined by a manufacturability learned model or a set of rules, the manufacturability learned model being a model on which machine learning has been performed by using the result of the thermodynamic calculation performed on the predetermined composition and the manufacturability indicating whether the material having the predetermined composition is manufacturable as training data, and the set of rules being for deriving the manufacturability from the result of the thermodynamic calculation performed on the predetermined composition.

4. The material search apparatus as claimed in any one of claims 1 to 3, wherein the composition obtaining unit further obtains a candidate manufacturing condition, and the material search apparatus comprises:
a property prediction unit configured to predict a property of a material having the candidate composition to be manufactured under the candidate manufacturing condition based on a correspondence relation between a predetermined composition and a predetermined manufacturing condition, and a property of a material having the predetermined composition that is manufactured under the predetermined manufacturing condition;
a required property obtaining unit configured to obtain a required property for the material having the candidate composition to be manufactured under the candidate manufacturing condition; and
a property determining unit configured to determine whether the predicted property meets the obtained required property.

5. The material search apparatus as claimed in claim 4, wherein the correspondence relation between the predetermined composition and the predetermined manufacturing condition, and the property of the material having the predetermined composition that is manufactured under the predetermined manufacturing condition is defined by a property prediction learned model or a set of rules based on statistical analysis, the property prediction learned model being a model on which machine learning has been performed by using the predetermined composition and the predetermined manufacturing condition, and the property of the material having the predetermined composition that is manufactured under the predetermined manufacturing condition, as training data.

6. The material search apparatus as claimed in claim 4, wherein, with respect to a composition used when the property determining unit determines that the predicted property meets the required property, the manufacturing determining unit determines whether a material having the composition is manufacturable.

7. The material search apparatus as claimed in claim 4,
wherein, with respect to the composition determined by the manufacturing determining unit to be the manufacturable material, the property determining unit determines whether the predicted property meets the required property, and
wherein the output unit outputs a composition that is determined to be the manufacturable material and determined to satisfy the required property.

8. The material search apparatus as claimed in claim 4,
wherein determining whether the candidate composition is the manufacturable material and determining whether the predicted property meets the required property are performed in parallel, and
wherein the output unit outputs a composition that is determined to be the manufacturable material and determined to satisfy the required property.

9. A method comprising:
a step of obtaining a candidate composition;
a step of performing thermodynamic calculation on the candidate composition;
a step of determining whether the candidate composition is a manufacturable material based on a correspondence relation between a result of the thermodynamic calculation performed on a predetermined composition and manufacturability indicating whether a material having the predetermined composition is manufacturable; and
a step of outputting a composition that is determined to be the manufacturable material.

10. A program for causing a computer to function as
a composition obtaining unit configured to obtain a candidate composition,
a thermodynamic calculation unit configured to perform thermodynamic calculation on the candidate composition,
a manufacturing determining unit configured to determine whether the candidate composition is a manufacturable material based on a correspondence relation between a result of the thermodynamic calculation performed on a predetermined composition and manufacturability indicating whether a material having the predetermined composition is manufacturable, and
an output unit configured to output a composition that is determined to be the manufacturable material.
